# FASCICULE DE BREVET EUROPEEN

(11) **EP 1 779 896 B1**
(45) Date de publication et mention de la délivrance du brevet: **01.08.2012**
(21) Numéro de dépôt: 06291669.7
(22) Date de dépôt: 26.10.2006
(51) Int. Cl.: A61Q 5/06, A61K 8/81, A61K 8/41, A61K 8/86, A61K 8/46

(54) **Composition cosmétique comprenant au moins un polymère fixant particulier et au moins un tensioactif ionique ou non ionique**
Kosmetische Zusammensetzung enthaltend mindestens ein fixierendes Polymer und eine ionische oder nicht ioniche Tenside
Cosmetic composition comprising at least a fixing polymer and an ionic or non ionic surfactant

(30) Priorité: 28.10.2005 FR 0511093
(43) Date de publication de la demande: 02.05.2007
(73) Titulaire: L'Oréal, 75008 Paris (FR)
(72) Inventeur: Laurent, Ludivine, 92400 Courbevoie (FR); Combarieu, Marie-Claude, 95230 Soisy sous Montmorency (FR)
(74) Mandataire: Zapalowicz, Francis

(56) Documents cités:
- EP-A- 1 205 175
- WO-A-2005/095479
- US-A- 5 686 067
- US-B1- 6 451 299

## Description

La présente invention concerne une composition cosmétique selon la revendication 1 pour le traitement des fibres kératiniques, en particulier des fibres kératiniques humaines telles que les cheveux, comprenant au moins un polymère fixant particulier et au moins un tensioactif cationique, une utilisation de cette composition pour la mise en forme et/ou le maintien de la coiffure ainsi qu'un procédé de traitement cosmétique la mettant en oeuvre.

Dans le domaine du coiffage, en particulier parmi les produits capillaires destinés à la mise en forme et/ou au maintien de la coiffure, les compositions capillaires sont généralement constituées d'une solution le plus souvent alcoolique ou aqueuse et d'un ou de plusieurs polymères fixants en mélange avec divers adjuvants cosmétiques.

Ces compositions peuvent se présenter sous forme de gels, de lotions ou de mousses capillaires qui sont généralement appliquées sur des cheveux mouillées avant d'effectuer un brushing ou un séchage.

En particulier, les gels capillaires sont notamment constitués d'un ou de plusieurs polymères épaississants ou agents gélifiants en association avec un ou plusieurs polymères fixants qui ont le plus souvent pour fonction de former un film à la surface des fibres kératiniques à fixer afin de réaliser des soudures entre celles-ci.

Cependant, l'application de ces gels capillaires conduit, plus ou moins rapidement, à la formation de résidus inesthétiques sur les cheveux qui sont dus à la fragilité ou à la friabilité dans le temps du film responsable de la fixation avec désagrégation partielle des soudures.

Par ailleurs, les gels capillaires sont difficilement diffusables sous forme de sprays dans des dispositifs aérosols ou dans des flacons pompes.

Il existe donc un réel besoin de trouver des compositions cosmétiques, notamment pour le coiffage, qui permettent de minimiser la formation de résidus inesthétiques tout en conférant à la chevelure un niveau élevé de fixation pour obtenir une mise en forme et/ou un maintien de la coiffure satisfaisant.

Le document US-A- 5 686 067 divulgue des sprays de fixation de la coiffure à base de polymère fixant selon la présente demande. Ces compositions peuvent éventuellement comprendre des tensioactifs. Ce document n'en précise pas la nature.

De manière surprenante et avantageuse, la Demanderesse vient de découvrir qu'en associant un polymère fixant particulier et un tensioactif cationique, il était possible notamment de minimiser la formation des résidus sur la chevelure et d'obtenir des propriétés cosmétiques satisfaisantes.

Une telle association permet également de conférer aux gels de coiffage une facilité de préhension ainsi qu'une texture de gel satisfaisante sans qu'il ne soit nécessaire d'ajouter un polymère épaississant ou un agent gélifiant.

En outre, les gels obtenus sont plus aisément sprayables c'est-à-dire diffusables sous forme de spray.

La présente invention a donc notamment pour objet une composition cosmétique pour le traitement des fibres, en particulier des fibres kératiniques humaines telles que les cheveux, comprenant une telle association.

Un autre objet de la présente invention consiste en une utilisation de la composition selon l'invention pour la mise en forme et/ou le maintien de la coiffure.

L'invention concerne également un dispositif aérosol comprenant une composition cosmétique selon l'invention.

Un autre objet encore est un procédé de traitement cosmétique mettant en oeuvre la composition selon l'invention

D'autres objets, caractéristiques, aspects et avantages de l'invention apparaîtront encore plus clairement à la lecture de la description et des exemples qui suivent.

Selon l'invention, la composition cosmétique pour le traitement des fibres kératiniques, en particulier des fibres kératiniques humaines telles que les cheveux, comprend, dans un milieu cosmétiquement acceptable :
- au moins un polymère fixant anionique comprenant au moins en tant que monomères un vinyl lactame, un acide carboxylique à instauration éthylénique et un acrylate ou un méthacrylate d'alkyle, la portion alkyle comportant au moins 6 atomes de carbone, et
- au moins un tensioactif cationique ; ladite composition présentant une viscosité strictement supérieure à 2 poise à 25°C (200 cps; 0,2 Pa·s) à un taux de cisaillement de 1s⁻¹.

La viscosité de la composition cosmétique peut être déterminée avec un rhéomètre de type RS600 de THERMOELECTRON.

La viscosité de la composition cosmétique conforme à l'invention est telle qu'il n'est pas nécessaire d'ajouter un polymère épaississant ou un agent gélifiant.

Le ou les tensioactifs ioniques utilisés dans la composition cosmétique sont des tensioactifs cationiques.

A titre d'exemple de tensioactifs cationiques utilisables dans la composition cosmétique, on peut notamment citer les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (I) suivante: 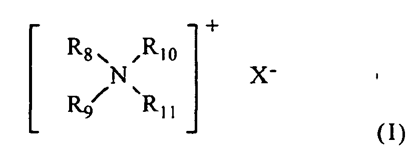
dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes.

Les radicaux aliphatiques sont par exemple choisis parmi les radicaux alkyle, alcoxy, polyoxyalkylène (C₂-C₆), alkylamide, alkyl(C₁₂₋ C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (II) suivante : 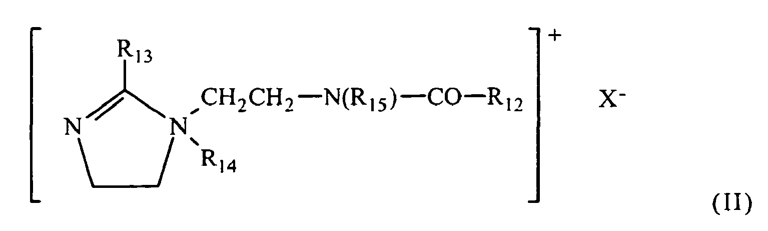
dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (III) : 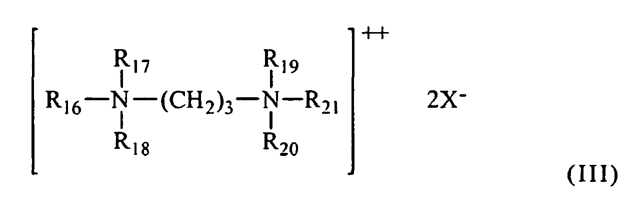 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propane suif diammonium;
   - les sels d'ammonium quaternaire contenant au moins une fonction ester, tels que ceux de formule (IV) suivante: 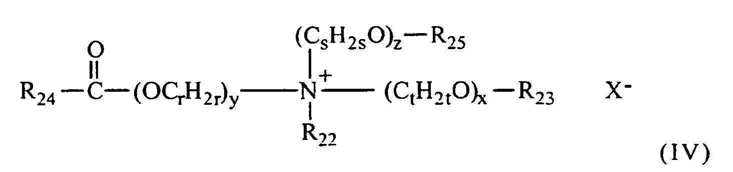
dans laquelle :
R₂₂ est choisi parmi les radicaux alkyles en C₁-C₆ et les radicaux hydroxyalkyles ou dihydroxyalkyles en C₁-C₆;
R₂₃ est choisi parmi :
   - le radical 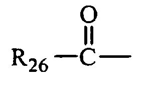
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 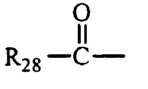
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10;
X⁻ est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 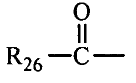
   - les radicaux méthyle, éthyle ou hydrocarbonés en C₁₄-C₂₂,
   - l'atome d'hydrogène ;
- R₂₅ est choisi parmi :
   - le radical 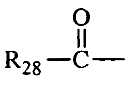
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (I), on préfère d'une part, les chlorures de tétraalkylammonium comme, par exemple, les chlorures de dialkyldiméthylammonium ou d'alkyltrimé thylammonium dans lesquels le radical alkyle comporte environ de 12 à 22 atomes de carbone, en particulier les chlorures de béhényltriméthyl ammonium, de distéaryldiméthylammonium, de cétyltriméthylammo nium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans la composition de l'invention sont choisis parmi les sels d'ammonium quaternaire, et en particulier parmi le chlorure de cétyltriméthyl ammonium, le chlorure de béhényltriméthylammonium et le chlorure de palmitylamidopropyltriméthylammonium.

Le ou les tensioactifs cationiques sont présents à une concentration allant de 0,01 à 20 % en poids, de préférence à une concentration allant de 0,05 à 10 % en poids, et encore plus préférentiellement à une concentration allant de 0,1 à 5 % en poids, par rapport au poids total de la composition.

Par polymère fixant, on entend au sens de la présente invention tout polymère permettant de conférer une forme ou de maintenir une forme ou une coiffure donnée.

L'acide carboxylique à insaturation éthylénique est choisi de préférence parmi l'acide acrylique, l'acide méthacrylique, l'acide itaconique ou l'acide crotonique.

De préférence l'acide carboxylique à insaturation éthylénique est l'acide acrylique ou méthacrylique. Encore plus préférentiellement, cet acide carboxylique à insaturation éthylénique est l'acide acrylique.

Le vinyl lactame est en particulier la vinyl pyrrolidone.

L'acrylate ou le méthacrylate d'alkyle comportant au moins 6 atomes de carbone, est de préférence un ester acrylique ou méthacrylique comprenant un radical alkyle comportant de 8 à 18 atomes de carbone.

Les radicaux alkyle sont choisis de préférence parmi les radicaux 2-éthyl hexyle, octyle, lauryle et stéaryle.

Ces polymères peuvent être éventuellement réticulés.

Les polymères fixants anioniques utilisables dans la composition selon l'invention peuvent être préparés par exemple selon le procédé décrit dans le brevet US 5 015708.

Les polymères fixants anioniques utilisables dans la composition selon l'invention comprennent, de préférence, en tant que monomères de la vinylpyrrolidone, de l'acide acrylique et du méthacrylate de lauryle.

Un polymère particulièrement préféré est le polymère commercialisé sous la dénomination « Acrylidone LM» par la société ISP qui est un terpolymère de vinylpyrrolidone (68%) / d'acide acrylique (23%) et de méthacrylate de lauryle (9%), les pourcentages en poids de chacun des monomères étant calculés par rapport au poids total du polymère fixant anionique.

Le(s) polymère(s) fixant(s) anionique(s) à base de vinyllactame, d'acide à insaturation éthylénique et d'acrylate ou de méthacrylate d'alkyle est ou sont notamment présent(s) dans la composition à une concentration allant de 0,05 à 30% en poids, de préférence à une concentration allant de 0,1 à 20% en poids, et encore plus préférentiellement à une concentration allant de 0,5 à 10% en poids, par rapport au poids total de la composition.

De préférence, le rapport pondéral entre le ou les polymères fixants anioniques de l'invention et le ou les tensioactifs cationiques de l'invention est supérieur à 1.

Dans un mode de réalisation préférée, la composition cosmétique comprend un polymère fixant à base de vinylpyrrolidone, d'acide acrylique et de méthacrylate de lauryle présent à une concentration inférieure à 3,5 % en poids et un tensioactif cationique présent à une concentration inférieure à 1 % en poids, les pourcentages étant calculés en poids par rapport au poids total de la composition cosmétique et ladite composition étant conditionnée dans un flacon pompe ou dans un dispositif aérosol.

Ces compositions particulières qui sont conditionnées dans ces dispositifs particuliers conduisent à des sprays de hautes qualités.

De manière générale, la composition cosmétique présente une viscosité comprise 2 et 1000 poise à 25°C (200 et 100000 cps), de préférence entre 5 et 500 poise à 25°C (500 et 50000 cps; 0,2 et 100 Pa·s), et encore plus préférentiellement entre 8 et 300 poise à 25°C (800 et 30000 cps; 0,8 et 30 Pa·s) à un taux de cisaillement de 1s⁻¹ mesurée à l'aide du rhéomètre RS600 de THERMOELECTRON.

Dans un mode de réalisation particulier de l'invention, les compositions de l'invention sont exemptes de polymères épaississants additionnels.

La composition cosmétique selon l'invention peut comprendre en outre au moins un adjuvant cosmétique parmi les polymères fixants autre que ceux de l'invention, les silicones sous forme soluble, dispersées, micro ou nano-dispersées, les agents épaississants non polymériques, des polymères épaississants ou agents gélifiants non sulfonés, les agents tensioactifs cationiques, anioniques, amphotères, non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les agents réducteurs ou antioxydants, les agents oxydants.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu aqueux, alcoolique ou hydroalcoolique. Ainsi le milieu peut notamment être constitué uniquement par de l'eau ou de l'alcool ou par un mélange d'eau et d'un ou de plusieurs solvants cosmétiquement acceptables tels que les alcools inférieurs en C₁-C₄, les polyols, les monoéthers de polyols et leurs mélanges.

Les compositions conformes à l'invention se présentent généralement sous forme de gel et peuvent être utilisées en application rincée ou non.

Ces compositions peuvent être conditionnées dans un vaporisateur, un flacon pompe ou dans un dispositif aérosol usuel en cosmétique.

Les agents propulseurs utilisés dans les systèmes aérosols selon l'invention peuvent être choisis parmi l'air, l'azote, le gaz carbonique, le diméthyléther, les alcanes en C₃ à C₅, le 1,1-difluoroéthane et leurs mélanges.

La présente invention concerne également un dispositif aérosol comprenant la composition selon l'invention ainsi qu'un moyen de distribution de cette composition.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou un éventuel séchage. De préférence, le mode d'application est un mode non rincé.

La présente invention concerne également l'utilisation d'une composition cosmétique pour la mise en forme et/ou le maintien de la coiffure.

Les exemples suivants sont donnés à titre illustratif de la présente invention.

### EXEMPLES

On prépare le gel de coiffage (B) selon l'invention à partir des composés suivants :

### Composition (B)

- Terpolymère vinylpyrrolidone/acide acrylique/ méthacrylate de lauryle, proposé par la société ISP sous la dénomination Acrylidone LM 2 %
- Chlorure de béhényl triméthyl ammonium (Tensioactif cationique) 0,5 %
- Conservateurs qs
- Eau qsp 100 %

On obtient un gel de coiffage (C) ayant une viscosité de 16 poise (1600 cps) à 25°C mesurée au rhéomètre RS600 de THERMOELECTRON à un taux de cisaillement de 1s⁻¹.

Les pourcentages de chacun des composés dans le gel de coiffage selon l'invention sont calculés en poids par rapport au poids total de la composition.

Le gel de coiffage (B) obtenu est appliqué sur la chevelure.

Les performances de ce gel de coiffage ont été évaluées par des professionnels.

On constate que le gel de coiffage (B) selon l'invention permet, tout en ayant une bonne fixation, de minimiser la formation de résidus inesthétiques sur la chevelure.

Par ailleurs, on observe que lorsque le gel de coiffage (B) est conditionné dans un flacon pompe, on obtient un spray très satisfaisant.

## Revendications

1. Composition cosmétique pour le traitement des fibres kératiniques, en particulier des fibres kératiniques humaines telles que les cheveux, **caractérisée par le fait qu'**elle comprend, dans un milieu cosmétiquement acceptable,
- au moins un polymère fixant anionique comprenant au moins en tant que monomères un vinyl lactame, un acide carboxylique à insaturation éthylénique et un acrylate ou un méthacrylate d'alkyle, la portion alkyle comportant au moins 6 atomes de carbone, et
- au moins un tensioactif cationique ;
et qui présente une viscosité strictement supérieure à 0,2 Pa.s à 25°C à un taux de cisaillement de 1s⁻¹.

2. Composition cosmétique selon la revendication 1, **caractérisée par le fait que** les tensioactifs cationiques sont choisis parmi les sels d'amines grasses primaires, secondaires ou tertiaires, éventuellement polyoxyalkylénées, les sels d'ammonium quaternaire et leurs mélanges.

3. Composition cosmétique selon la revendication 2, **caractérisée par le fait que** les sels d'ammonium quaternaire sont choisis parmi:
- ceux qui présentent la formule générale (I) suivante : 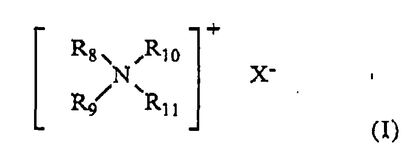 dans laquelle les radicaux R₈ à R₁₁, qui peuvent être identiques ou différents, représentent un radical aliphatique, linéaire ou ratifié, comportant de 1 à 30 atomes de carbone, ou un radical aromatique tel que aryle ou alkylaryle ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (II) : 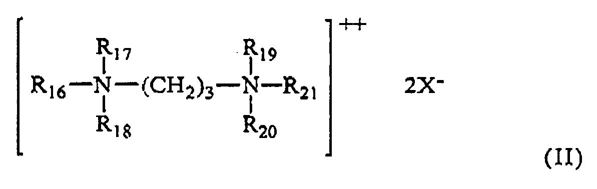 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates ;
- les sels d'ammonium quaternaire contenant au moins une fonction ester.

4. Composition cosmétique selon l'une quelconques des revendications précédentes, **caractérisée par le fait que** l'acide carboxylique insaturé est choisi parmi l'acide acrylique, l'acide méthacrylique, l'acide itaconique ou l'acide crotonique.

5. Composition selon la revendication 4, **caractérisée par le fait que** l'acide carboxylique insaturé est l'acide acrylique.

6. Composition cosmétique selon l'une quelconque des revendications précédentes, **caractérisée par le fait que** le vinyl lactame est la vinylpyrrolidone.

7. Composition cosmétique selon l'une quelconque des revendications précédentes, **caractérisée par le fait que** les esters acrylique ou méthacrylique comportent un radical alkyle comportant de 8 à 18 atomes de carbone.

8. Composition cosmétique selon la revendication 7, **caractérisée par le fait que** les radicaux alkyle des esters acrylique ou méthacrylique sont choisis parmi les radicaux 2-éthylhexyle, octyle, lauryle et stéaryle.

9. Composition cosmétique selon l'une quelconque des revendications précédentes, **caractérisée par le fait que** ledit polymère fixant anionique comprend en tant que monomères de la vinylpyrrolidone, de l'acide acrylique et du méthacrylate de lauryle.

10. Composition cosmétique selon l'une quelconque des revendications précédentes, **caractérisée par le fait que** ledit polymère fixant est présent à une concentration allant de 0,05 à 30 % en poids, de préférence à une concentration allant de 0,1 à 20 % en poids, et encore plus préférentiellement à une concentration allant de 0,5 à 10 % en poids, par rapport au poids total de la composition.

11. Composition cosmétique selon l'une quelconque des revendications précédentes, **caractérisée par le fait que** ledit ou lesdits tensioactifs cationiques sont presents à une concentration allant de 0,01 à 20 % en poids, de préférence à une concentration allant de 0,05 à 10 % en poids, et encore plus préférentiellement à une concentration allant de 0,1 à 5 % en poids, par rapport au poids total de la composition.

12. Composition cosmétique selon l'une quelconque des revendications précédentes, caractérisée jar le fait qu'elle présente une viscosité comprise entre 0,2 et 1 Pa.s à 25°C, de préférence entre 0,5 et 5 Pa.s à 25°C, et encore plus préférentiellement entre 0,8 et 3 Pa.s à 25°C à un taux de cisaillement de 1s⁻¹.

13. Composition cosmétique selon l'une quelconque des revendications précédentes, **caractérisée par le fait que** le rapport pondéral entre le ou les polymères fixants anioniques et le ou les tensioactifs cationiques est supérieur à 1.

14. Composition selon l'une quelconque des revendications précédentes, **caractérisée par le fait qu'**elle comprend en outre au moins un adjuvant cosmétique choisi parmi les polymères fixants autre que ceux de l'invention, les silicones sous forme soluble, dispersées, micro ou nano-dispersées, les agents épaississants non polymériques, des polymères épaississants ou agents gélifiants non sulfonés, les agents tensioactifs, anioniques, amphotères, non ioniques, les agents conditionneurs de type esters, les agents anti-mousse, les agents hydratants, les agents émollients, les plastifiants, les filtres solaires hydrosolubles et liposoluble, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anticorrosion, les corps gras tels que les huiles végétales, animales, minérales et synthétiques, les agents réducteurs ou antioxydants, les agents oxydants.

15. Composition cosmétique selon la revendication 14, **caractérisée par le fait que** le ou les adjuvants cosmétiques sont présents à une concentration allant de 0,001 à 50 % en poids par rapport au poids total de la composition.

16. Composition cosmétique selon l'une quelconque des revendications précédentes, **caractérisée par le fait que** le milieu cosmétiquement acceptable est un milieu aqueux, alcoolique ou hydroalcoolique.

17. Composition cosmétique selon la revendication 16, **caractérisée par le fait que** le milieu hydroalcoolique comprend les alcools inférieurs en C₁-C₄, les polyols, les monoéthers de polyols et leurs mélanges.

18. Composition cosmétique selon la revendication 17, **caractérisée par le fait que** l'alcool est de l'éthanol.

19. Composition cosmétique selon l'une quelconque des revendications précédentes, **caractérisée par le fait qu'**elle est exempte de polymères épaississants additionnels.

20. Composition cosmétique selon l'une quelconque des revendications précédentes, **caractérisée par le fait qu'**elle est conditionnée dans un vaporisateur, un flacon pompe ou un dispositif aérosol.

21. Composition cosmétique selon la revendication 20, **caractérisée par le fait qu'**elle est conditionnée dans un dispositif aérosol.

22. Composition cosmétique selon la revendication 21, **caractérisée par le fait qu'**elle comprend un agent propulseur choisi parmi l'air, l'azote, le gaz carbonique, le diméthyléther, les alcanes en C₃ à C₅, le 1,1-difluoroéthane et leurs mélanges.

23. Composition cosmétique selon la revendication 1, **caractérisée par le fait que** ledit polymère fixant anionique est un polymère à base de vinylpyrrolidone, d'acide acrylique et de méthacrylate de lauryle présent à une concentration inférieure à 3,5 % en poids et que ledit ou lesdits tensioactifs cationiques sont présent(s) à une concentration inférieure à 10 % en poids, par rapport au poids total de la composition, ladite composition étant conditionnée dans un flacon pompe ou dans un dispositif aérosol.

24. Dispositif aérosol formé par un récipient comprenant une composition selon l'une quelconque des revendications précédentes ainsi qu'un moyen de distribution de la composition.

25. Procédé de traitement cosmétique **caractérisée par le fait qu'**il comprend l'application d'une composition cosmétique selon l'une quelconque des revendications 1 à 23, en particulier sur les cheveux.

26. Procédé de traitement cosmétique selon la revendication 25, **caractérisée par le fait que** l'application de ladite composition n'est pas suivie par un rinçage.

27. Utilisation d'une composition cosmétique selon l'une quelconque des revendications 1 à 23 pour la mise en forme et/ou le maintien de la coiffure.

## Claims

1. Cosmetic composition for treating keratin fibres, especially human keratin fibres such as the hair, **characterized in that** it comprises, in a cosmetically acceptable medium,
- at least one anionic fixative polymer comprising at least as monomers a vinyl lactam, an ethylenically unsaturated carboxylic acid and an alkyl acrylate or methacrylate whose alkyl moiety contains at least 6 carbon atoms, and
- at least one cationic surfactant; and exhibits a viscosity strictly greater than 0.2 Pa.s at 25°C at a shear rate of 1 s⁻¹.

2. Cosmetic composition according to Claim 1, **characterized in that** the cationic surfactants are selected from the salts of optionally polyoxyalkylenated primary, secondary or tertiary fatty amines, quaternary ammonium salts and mixtures thereof.

3. Cosmetic composition according to Claim 2, **characterized in that** the quaternary ammonium salts are selected from:
- those having the general formula (I) below: 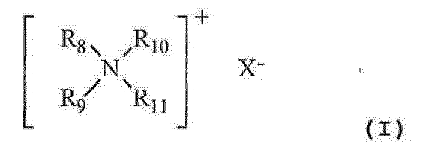 in which the radicals R₈ to R₁₁, which may be identical or different, represent a linear or branched aliphatic radical containing 1 to 30 carbon atoms, or an aromatic radical such as aryl or alkylaryl; X is an anion selected from the group of halides, phosphates, acetates, lactates, C₂-C₁₅ alkyl sulphates, alkylsulphonates and alkylarylsulphonates;
- quaternary ammonium salts of imidazoline;
- quaternary diammonium salts of formula (II): 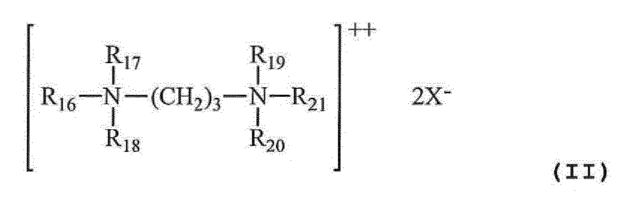 in which R₁₆ denotes an aliphatic radical containing approximately 16 to 30 carbon atoms, R₁₇, R₁₈, R₁₉, R₂₀ and R₂₁, which are identical or different, are selected from hydrogen and an alkyl radical containing 1 to 4 carbon atoms, and X is an anion selected from the group of halides, acetates, phosphates, nitrates and methyl sulphate;
- quaternary ammonium salts containing at least one ester function.

4. Cosmetic composition according to any one of the preceding claims, **characterized in that** the unsaturated carboxylic acid is selected from acrylic acid, methacrylic acid, itaconic acid and crotonic acid.

5. Composition according to Claim 4, **characterized in that** the unsaturated carboxylic acid is acrylic acid.

6. Cosmetic composition according to any one of the preceding claims, **characterized in that** the vinyl lactam is vinylpyrrolidone.

7. Cosmetic composition according to any one of the preceding claims, **characterized in that** the acrylic or methacrylic esters include an alkyl radical containing 8 to 18 carbon atoms.

8. Cosmetic composition according to Claim 7, **characterized in that** the alkyl radicals of the acrylic or methacrylic esters are selected from 2-ethylhexyl octyl, lauryl and stearyl radicals.

9. Cosmetic composition according to any one on the preceding claims, **characterized in that** the said anionic fixative polymer comprises as monomers vinylpyrrolidone, acrylic acid and lauryl methacrylate.

10. Cosmetic composition according to any one of the preceding claims, **characterized in that** the said fixative polymer is present at a concentration, ranging from 0.05% to 30% by weight, preferably at a concentration ranging from 0.1% to 20% by weight and more preferably at a concentration ranging from 0.5% to 10% by weight, relative to the total weight of the composition.

11. Cosmetic composition according to any one of the preceding claims, **characterized in that** the said cationic surfactant or surfactants are present at a concentration ranging from 0.01% to 20% by weight, preferably at a concentration ranging from 0.05% to 10% by weight and more preferably at a concentration ranging from 0.1% to 5% by weight, relative to the total weight of the composition.

12. Cosmetic composition according to any one of the preceding claims, **characterized in that** it exhibits a viscosity of between 0.2 and 1 Pa.s at 25°C, preferably between 0.5 and 5 Pa.s at 25°C and more preferably between 0.8 and 3 Pa.s at 25°C at a shear rate of 1 s⁻¹.

13. Cosmetic composition according to any one of the preceding claims, **characterized in that** the weight ratio between the anionic fixative polymer or polymers and the cationic surfactant or surfactants is greater than 1.

14. Composition according to any one of the preceding claims, **characterized in that** it further comprises at least one cosmetic adjuvant selected from fixative polymers other than those of the invention, dispersed, microdispersed or nanodispersed silicones in soluble form, non-polymeric thickeners, thickening polymers or gelling agents which are not sulphonated, anionic, amphoteric and nonionic surfactants, ester-type conditioning agents, antifoams, moisturizers, emollients, plasticizers, water-soluble and fat-soluble silicone or non-silicone sunscreens, permanent or temporary dyes, fragrances, peptizing agents, preservatives, ceramides and pseudoceramides, vitamins and provitamins, including panthenol, proteins, sequestrants, solubilizers, alkalifiers, anticorrosives, fatty substances such as vegetable, animal, mineral and synthetic oils, reducing agents or antioxidants, and oxidizing agents.

15. Cosmetic composition according to Claim 14, **characterized in that** the cosmetic adjuvant or adjuvants are present at a concentration ranging from 0.001% to 50% by weight, relative to the total weight of the composition.

16. Cosmetic composition according to any one of the preceding claims, **characterized in that** the cosmetically acceptable medium is an aqueous, alcoholic or aqueous-alcoholic medium.

17. Cosmetic composition according to Claim 16, **characterized in that** the aqueous-alcoholic medium comprises C₁-C₄ lower alcohols, polyols, polyol monoethers and mixtures thereof.

18. Cosmetic composition according to Claim 17, **characterized in that** the alcohol is ethanol.

19. Cosmetic composition according to any one of the preceding claims, **characterized in that** it is devoid of additional thickening polymers.

20. Cosmetic composition according to any one of the preceding claims, **characterized in that** it is packaged in a vaporizer, a pump dispenser bottle or an aerosol device.

21. Cosmetic composition according to Claim 20, **characterized in that** it is packaged in an aerosol device.

22. Cosmetic composition according to Claim 21, **characterized in that** it comprises a propellant selected from air, nitrogen, carbon dioxide, dimethyl ether, C₃ to C₅ alkanes, 1,1-difluoroethane and mixtures thereof.

23. Cosmetic composition according to Claim 1, **characterized in that** the said anionic fixative polymer is a polymer which is based on vinylpyrrolidone, acrylic acid and lauryl methacrylate and is present at a concentration of less than 3.5% by weight and **in that** the said cationic surfactant or surfactants are present at a concentration of less than 10% by weight, relative to the total weight of the composition, the said composition being packaged in a pump dispenser bottle or in an aerosol device.

24. Aerosol device formed by a container comprising a composition according to any one of the preceding claims and a means of distributing the composition.

25. Cosmetic treatment method **characterized in that** it comprises applying a cosmetic composition according to any one of Claims 1 to 23, in particular to the hair.

26. Cosmetic treatment method according to Claim 25, **characterized in that** the application of the said composition is not followed by a rinsing operation.

27. Use of a cosmetic composition according to any one of Claims 1 to 23 for shaping and/or retaining the hairstyle.

## Patentansprüche

1. Kosmetikzusammensetzung für die Behandlung von Keratinfasern, insbesondere menschlichen Keratinfasern wie dem Haar, **dadurch gekennzeichnet, dass** sie in einem kosmetisch unbedenklichen Medium Folgendes umfasst:
- mindestens ein anionisches fixierendes Polymer mit mindestens einem Vinyllactam, einer Carbonsäure mit ethylenischer Ungesättigtheit und ein Alkylacrylat oder
- methacrylat, wobei der Alkylteil mindestens 6 Kohlenstoffatome umfasst, als Monomere, sowie
- mindestens ein kationisches Tensid;
und die eine Viskosität von streng oberhalb von 0,2 Pa.s bei 25°C und einer Scherrate von 1 s⁻¹ aufweiset.

2. Kosmetikzusammensetzung nach Anspruch 1, **dadurch gekennzeichnet, dass** die kationischen Tenside aus den Salzen von gegebenenfalls polyoxyalkylenierten primären, sekundären oder tertiären Fettaminen, den quartären Ammoniumsalzen und ihren Mischungen ausgewählt sind.

3. Kosmetikzusammensetzung nach Anspruch 2, **dadurch gekennzeichnete dass** die quartären Ammoniumsalze aus Folgenden ausgewählt sind:
- denjenigen der folgenden allgemeinen Formel (I): 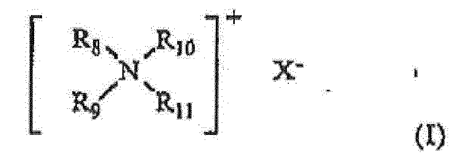 in der die Reste R₈ bis R₁₁, die gleich oder verschieden sein können, einen aliphatischen geradkettigen oder verzweigten Rest mit 1 bis 30 Kohlenstoffatomen oder einen aromatischen Rest wie Aryl oder Alkylaryl bedeuten; X ein Anion aus der Gruppe der Halogenide, Phosphate, Acetate, Lactate, Alkyl (C₂-C₆) sulfate und Alkyl- oder Alkylarylsulfonate bedeutet;
- den quartären Ammoniumsalzen von Imidazolin;
- den quartären Diammoniumsalzen der Formel (II): 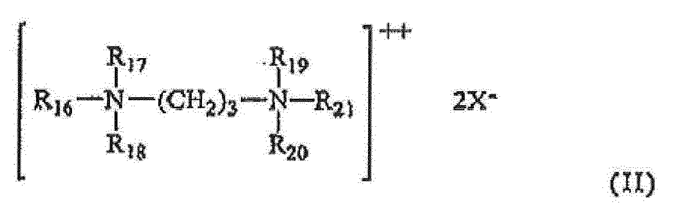 in der R₁₆ einen aliphatischen Rest mit ungefähr 16 bis 30 Kohlenstoffatomen bedeutet, R₁₇, R₁₈, R₁₉, R₂₀ und R₂₁, die gleich oder verschieden sind, unter einem Wasserstoff oder Alkylrest mit 1 bis 4 Kohlenstoffatomen ausgewählt sind und X ein Anion aus der Gruppe der Halogenide, Acetate, Phosphate, Nitrate und Methylsulfate bedeutet;
- den quartären Ammoniumsalzen, die mindestens eine Esterfunktion entfalten.

4. Kosmetikzusammensetzung nach einem der vorhergehenden Ansprüche, **dadurch gekennzeichnet, dass** die ungesättigte Carbonsäure unter Acrylsäure, Methacrylsäure, Itaconsäure oder Crotonsäure ausgewählt ist.

5. Zusammensetzung nach Anspruch 4, **dadurch gekennzeichnet, dass** es sich bei der ungesättigten Carbonsäure um Acrylsäure handelt.

6. Kosmetikzusamriensetzung nach einem der vorhergehenden Ansprüche, **dadurch gekennzeichnet, dass** es sich bei dem Vinyllactam um Vinylpyrrolidon handelt.

7. Kosmetikzusammensetzung nach einem der vorhergehenden Ansprüche, **dadurch gekennzeichnet, dass** die Acrylsäure- oder Methacrylsäureester einen Alkylrest mit 8 bis 18 Kohlenstoffatomen umfassen.

8. Kosmetikzusammensetzung nach Anspruch 7, **dadurch gekennzeichnet, dass** die Alkylreste der Acrylsäure- oder Methacrylsäureester aus der Reihe der 2-Ethylhexyl-, Octyl-, Lauryl- und Stearylreste ausgewählt sind.

9. Kosmetikzusammensetzung nach einem der vorhergehenden Ansprüche, **dadurch gekennzeichnet, dass** das anionische fixierende Polymer Vinylpyrrolidon, Acrylsäure und Laurylmethacrylat als Monomere enthält.

10. Kosmetikzusammensetzung nach einem der vorhergehenden Ansprüche, **dadurch gekennzeichnet, dass** das fixierende Polymer in einer Konzentration von 0,05 bis 30 Gew.-%, vorzugsweise in einer Konzentration von 0,1 bis 20 Gew.-% und noch stärker bevorzugt in einer Konzentration von 0,5 bis 10 Ges.-% in Bezug auf das Gesamtgewicht der Zusammensetzung vorliegt.

11. Kosmetikzusammensetzung nach einem der vorhergehenden Ansprüche, **dadurch gekennzeichnet, dass** das/die kationische(n) Tensid(e) in einer Konzentration von 0,01 bis 20 Ges.-%, vorzugsweise in einer Konzentration von 0,05 bis 10 Ges.-% und noch stärker bevorzugt in einer Konzentration von 0,1 bis 5 Gew.-% in Bezug auf das Gesamtgewicht der Zusammensetzung vorliegen.

12. Kosmetikzusammensetzung nach einem der vorhergehenden Ansprüche, **dadurch gekennzeichnet**, sie eine Viskosität zwischen 0,2 und 1 Pa.s bei 25°C, vorzugsweise zwischen 0,5 und 5 Pa.s bei 25°C und noch stärker bevorzugt zwischen 0,8 und 3 Pa.s bei 25°C bei einer Scherrate von 1 s⁻¹ aufweist.

13. Kosmetikzusammensetzung nach einem der vorhergehenden Ansprüche, **dadurch gekennzeichnet, dass** das Gewichtsverhältnis zwischen dem anionischen fixierenden Polymer bzw. den anionischen fixierenden Polymeren und dem oder den kationischen Tensid(en) größer als 1 ist.

14. Zusammensetzung nach einem der vorhergehenden Ansprüche, **dadurch gekennzeichnet, dass** sie weiterhin mindestens ein kosmetisches Hilfsmittel aus der Reihe fixierende Polymere, bei denen es sich nicht um diejenigen der Erfindung handelt, Silikone in löslicher Form, dispergierte, mikro- oder nanodispergierte Silikone, nichtpolymere Verdickungsmittel, verdickende Polymere oder nichtsulfonierte Gelbildner nichtsulfonierte Gelbildner verdickende Polymere, anionische, amphotere, nichtionische Tenside, Konditionierungsmittel des Estertyps, Antischaummittel, feuchtigkeitsspendende Mittel, zartmachende Mittel, Weichmacher, gegebenenfalls silikonhaltige wasserlösliche und fettlösliche Sonnenschutzfilter, Permanentfarbstoffe oder nichtpermanente Farbstoffe, Parfüms, Peptisatoren, Konservierungsmittel, Ceramide und Pseudoceramide, Vitamine und Provitamine, darunter Panthenol, Proteine, Sequestriermittel, Lösungsmittel, Alkalinisierungsmittel, Korrosionsschutzmittel, Fettkörper wie pflanzliche, tierische, mineralische und synthetische Öle, Reduktionsmittel oder Antioxidationsmittel und Oxidationsmittel umfasst.

15. Kosmetikzusammensetzung nach Anspruch 14, **dadurch gekennzeichnet, dass** der/die kosmetische(n) Hilfstoff(e) in einer Konzentration von 0,001 bis 50 Ges.-% in Bezug auf das Gesamtgewicht der Zusammensetzung vorliegen.

16. Kosmetikzusammensetzung nach einem der vorhergehenden Ansprüche, **dadurch gekennzeichnet, dass** es sich bei dem kosmetisch unbedenklichen Medium um ein wässriges, alkoholisches oder wässrig-alkoholisches Medium handelt.

17. Kosmetikzusammensetzung nach Anspruch 16, **dadurch gekennzeichnet, dass** das wässrig-alkoholische Medium niedere C₁-C₄-Alkohole, Polyole, Polyolmonoether und ihre Mischungen umfasst.

18. Kosmetikzusammensetzung nach Anspruch 17, **dadurch gekennzeichnet, dass** es sich bei dem Alkohol um Ethanol handelt.

19. Kosmetikzusammensetzung nach einem der vorhergehenden Ansprüche, **dadurch gekennzeichnet, dass** sie frei von zusätzlichen verdickenden Polymeren ist.

20. Kosmetikzusammensetzung nach einem der vorhergehenden Ansprüche, **dadurch gekennzeichnet, dass** sie in einem Verdampfer, einer Pumpflasche oder einer Aerosolvorrichtung verpackt ist.

21. Kosmetikzusammensetzung nach Anspruch 20, **dadurch gekennzeichnet, dass** sie in einer Aerosolvorrichtung verpackt ist.

22. Kosmetikzusammensetzung nach Anspruch 21, **dadurch gekennzeichnet, dass** sie ein Treibmittel aus der Reihe Luft, Stickstoff, Kohlendioxid, Dimethylether, C₃- bis C₅-Alkane, 1,1-Di-fluorethan und ihre Mischungen umfasst.

23. Kosmetikzusammensetzung nach Anspruch 1, **dadurch gekennzeichnet, dass** es sich bei dem anionischen fixierenden Polymer um ein Polymer auf Basis von Vinylpyrrolidon, Acrylsäure und Laurylmethacrylat in einer Konzentration von unter 3,5 Gew.-% handelt und dass das/die kationische(n) Tensid(e) in einer Konzentration von unter 10 Gew.-% in Bezug auf das Gesamtgewicht der Zusammensetzung vorliegen, wobei die Zusammensetzung in einer Pumpflasche oder in einer Aerosolvorrichtung verpackt ist.

24. Aerosolvorrichtung, die aus einem Behältnis, das eine Zusammensetzung nach einem der vorhergehenden Ansprüche enthält, sowie einem Mittel zum Verteilen der Zusammensetzung gebildet wird.

25. Kosmetisches Behandlungsverfahren, **dadurch gekennzeichnet, dass** es das Ausbringen einer Kosmetikzusammensetzung nach einem der Ansprüche 1 bis 23, insbesondere auf das Haar, umfasst.

26. Kosmetisches Behanllungsverfahren nach Anspruch 25, **dadurch gekennzeichnet, dass** nach der Ausbringung der Zusammensetzung nicht gespült wird.

27. Verwendung einer Kosmetikzusammensetzung nach einem der Ansprüche 1 bis 23 zum Formen und/oder Halten der Frisur.
